# EUROPEAN PATENT SPECIFICATION

(11) **EP 1 459 070 B1**
(45) Date of publication and mention of the grant of the patent: **13.06.2012**
(21) Application number: 02779771.1
(22) Date of filing: 27.11.2002
(51) Int. Cl.: G01N 33/574

(54) **METHODS FOR DIAGNOSIS AND TREATMENT OF EPITHELIAL-DERIVED CANCERS, SUCH AS COLORECTAL CANCERS AND KIDNEY CANCERS**
VERFAHREN FÜR DIAGNOSE UND BEHANDLUNG VON EPITHELKREBS WIE KOLOREKTALER ODER NIERENKREBS
METHODES PERMETTANT DE DIAGNOSTIQUER ET DE TRAITER DES CANCERS EPITHELIAUX TELS QUE LES CANCERS COLORECTAUX ET RENAUX

(30) Priority: 27.11.2001 US 333436 P
(43) Date of publication of application: 22.09.2004
(73) Proprietor: UCB Pharma S.A., 1070 Brussels (BE)
(72) Inventor: Terrett, J.A., Oxford GlycoSciences (UK) Ltd, Abingdon, Oxfordshire OX14 4RY (GB)
(74) Representative: Thompson, John
(86) International application number: PCT/GB2002/005335
(87) International publication number: WO 2003/046581

(56) References cited:
- WO-A-01/94629
- WISCHHUSEN J. ET AL.: "Identification of CD70-mediated apoptosis of immune effector cells as a novel immune escape pathway of human glioblastoma." CANCER RESEARCH, vol. 62, 2002, pages 2592-2599, XP002260384
- HISHIMA T. ET AL.: "CD70 expression in thymic carcinoma" AMERICAN JOURNAL OF SURGICAL PATHOLOGY, vol. 24, no. 5, 2000, pages 742-746, XP009020516
- GOODWIN R.G. ET AL.: "MOLECULAR AND BIOLOGICAL CHARACTERIZATION OF A LIGAND FOR CD27 DEFINES A NEW FAMILY OF CYTOKINES WITH HOMOLOGY TO TUMOR NECROSIS FACTOR" CELL, CELL PRESS, CAMBRIDGE, NA, US, vol. 73, 7 May 1993 (1993-05-07), pages 447-456, XP002016675 ISSN: 0092-8674
- WEINER L.M. AND ADAMS G.P.: "New approaches to antibody therapy" ONCOGENE, vol. 19, 2000, pages 6144-6151, XP002268541

### SEQUENCE LISTING

<110> Terrett, Jonathan Alexander Oxford GlycoSciences (UK) Ltd.
<120> Methods for Diagnosis and Treatment of Epithelial-Derived Cancers
<130> P0134-WO01
<150> US 60/333,436
   <151> 2001-11-27
<160> 4
<170> FastSEQ for Windows Version 4.0
<210> 1
   <211> 926
   <212> DNA
   <213> homo sapiens
<400> 1
   ccagagaggg gcaggcttgt cccctgacag gttgaagcaa gtagacgccc aggagccccg  60
   ggagggggct gcagtttcct tccttccttc tcggcagcgc tccgcgcccc catcgcccct 120
   cctgcgctag cggaggtgat cgccgcggcg atgccggagg agggttcggg ctgctcggtg 180
   cggcgcaggc cctatgggtg cgtcctgcgg gctgctttgg tcccattggt cgcgggcttg 240
   gtgatctgcc tcgtggtgtg catccagcgc ttcgcacagg ctcagcagca gctgccgctc 300
   gagtcacttg ggtgggacgt agctgagctg cagctgaatc acacaggacc tcagcaggac 360
   cccaggctat actggcaggg gggcccagca ctgggccgct ccttcctgca tggaccagag 420
   ctggacaagg ggcagctacg tatccatcgt gatggcatct acatggtaca catccaggtg 480
   acgctggcca tctgctcctc cacgacggcc tccaggcacc accccaccac cctggccgtg 540
   ggaatctgct ctcccgcctc ccgtagcatc agcctgctgc gtctcagctt ccaccaaggt 600
   tgtaccattg tctcccagcg cctgacgccc ctggcccgag gggacacact ctgcaccaac 660
   ctcactggga cacttttgcc ttcccgaaac actgatgaga ccttctttgg agtgcagtgg 720
   gtgcgcccct gaccactgct gctgattagg gttttttaaa ttttatttta ttttatttaa 780
   gttcaagaga aaaagtgtac acacaggggc caccccgggt tggggtggga gtgtggtggg 840
   gggtagtttg tggcaggaca agagaaggca ttgagctttt tctttcattt tcctattaaa 900
   aaatacaaaa atcaaaacaa aaaaaa                                     926
<210> 2
   <211> 193
   <212> PRT
   <213> homo sapiens
<400> 2
<210> 3
   <211> 22
   <212> DNA
   <213> Artificial Sequence
<220>
   <223> primer
<400> 3
   gctgctttgg tcccattggt cg 22
<210> 4
   <211> 22
   <212> DNA
   <213> Artificial Sequence
<220>
   <223> primer
<400> 4
   gaggtcctgt gtgattcagc tg 22

## Description

### BACKGROUND OF THE INVENTION

### Field of the invention.

The present invention relates to the use of a polypeptide (CD27L) for diagnosis of epithelial-related cancers, in particular kidney cancer e.g. renal cell cancer and colorectal cancer, e.g. colon cancers, as well as in methods of treatment of such cancers.

Treatment of most cancer types is usually via surgery, chemotherapy, radiotherapy or biological therapy. However, some tumours become refractory to such treatments, as the cancer cells develop resistance to chemotherapy drugs or lose their hormone sensitivity, leading to recurrent or metastatic disease which is often incurable. More recently, attention has focused on the development of immunological therapies (Green et al. (2000) Cancer Treat. Rev. 26, 269-286; Davis (2000) Immunol. Cell Biol. 78, 179-195; Knuth et al. (2000) Cancer Chemother Pharmacol. 46, S46-51; Shiku et al. (2000) Cancer Chemother. Pharmacol. 46, S77-82; Saffran et al. (1999) Cancer Metastasis Rev. 18, 437-449), such as cancer vaccines and monoclonal antibodies (mAbs), as a means of initiating and targeting a host immune response against tumour cells. In 1998, the FDA approved the use of herceptin (Stebbing et al. (2000) Cancer Treat. Rev. 26, 287-290; Dillman (1999) Cancer Biother. Radiopharm. 14, 5-10 ; Miller et al. (1999) Invest. New Drugs 17,417-427), a mAb that recognises the erbB2/HER2-neu receptor protein, as a treatment for metastatic breast cancer. In combination with chemotherapy, herceptin has been shown to prolong the time to disease progression, when compared to patients receiving chemotherapy alone (Baselga et al. (1998) Cancer Res. 58, 2825-2831). The identification of other suitable targets or antigens for immunotherapy of other cancers, for example epithelial-derived cancers, has become increasingly important.

### Kidney Cancer

There are three main types of kidney cancer - renal cell cancer that develops in the lining of the renal tubules which filter blood, Wilm's Tumor, found mainly in children under 5 years, and transitional cell cancer of the renal pelvis and/or ureter, that develops in the lining of the bladder, ureters or renal pelvis. Respectively they account for approximately 80%, 5% and 7% of all kidney cancer cases.

As kidney cancer grows, it may invade organs near the kidney, such as the liver, colon, or pancreas. When kidney cancer spreads, cancer cells may appear in the lymph nodes. For this reason, lymph nodes near the kidney may be removed during surgery. Kidney cancer may spread and form new cancers, most often in the bones or lungs.

Surgery is the main treatment for kidney cancer. The aim of surgery is to remove all or as much of the cancer as is possible and to 'stage' the cancer accurately so that the need for any further treatment can be assessed. In radical nephrectomy, the whole kidney along with the cancer is removed. The adrenal gland, which is attached to the kidney, is also removed along with the fatty tissue surrounding the kidney. Nearby lymph nodes are also removed as this helps the doctors decide which stage the cancer is. In a partial nephrectomy, only the part of the kidney that contains the cancer is removed and is less commonly performed. In some circumstances, removal of secondary metastases may be done to relieve symptoms such as pain. However, it does not usually help in terms of prognosis and will only be attempted if the cancer is easy to get to and surgery can be performed without causing any serious side effects. Arterial embolisation is a procedure done to block the artery of the kidney containing the cancer. This procedure may be done to control a primary tumour which surgery cannot remove or occasionally prior to an operation to make surgery easier.

Radiotherapy is sometimes used instead of surgery for patients who are too ill to undergo a major operation. In some circumstances it may be used to help with symptoms that arise as a result of recurrent or advanced cancers. However, renal cell kidney cancers are not particularly sensitive to radiotherapy and its use is not routine because studies have not shown that it improves prognosis.

Immunotherapeutic treatment most commonly uses cytokines interleukin-2 and interferon-alpha for the treatment of advanced (metastatic) kidney cancer.

### Colorectal Cancer

Excluding skin cancers, colorectal cancer is the third most common cancer diagnosed in men and women in the United States. Surgery is the main treatment for colorectal cancer. Radiation therapy is often used after surgery to kill unremoved deposits and to prevent local recurrences. Adjuvant chemotherapy may also be used, with drugs such as Fluorouracil (5-FU), optionally with leucovorin or levamisole, and Irinotecan (CPT-11). There are no generally approved immunotherapeutic drugs for the treatment of colorectal cancer, despite the fact that immunotherapy may offer the greatest potential after surgical resection in the adjuvant setting. Edrecolomab (monoclonal antibody 17-1A, or Panorex), however, is an adjunctive therapy for colorectal cancer which is in clinical trials in the UK and the US, and which has already been approved in Germany. Identification of new suitable targets or antigens for immunotherapy of colorectal cancer is therefore highly important.

### BRIEF SUMMARY OF THE INVENTION

The present invention is based on the finding that the protein, CD27L, exhibits elevated expression in epithelial-derived cancers, especially kidney cancer and colorectal cancer. The elevated expression of CD27L is useful diagnostically as well as a target for therapeutic treatment. CD27L is a ligand that binds the cytokine CD27 receptor found on the surface of human T and B lymphocytes (US 5,573,924; US 5,716,805; WO 94/05691; Goodwin et al. (1993) Cell 73(3):447-456). It was originally identified as CD70 see Hintzen et al Int Immunol, (1994), 6(3), pp477-80. A study published in 1991 used monoclonal antibodies to various activation antigens including CD70/CD27L and identified that CD70/CD27L had a high expression on activated B and T cells (Paloczi K et al., (1991), Heamatologica, 24(2), pp83-90). To avoid confusion the protein will be referred to herein as CD27L.

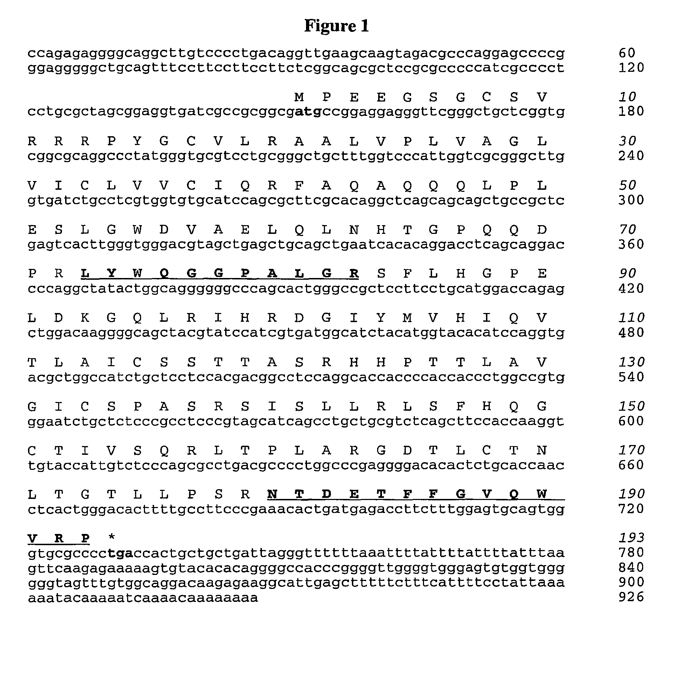

The present invention provides a method of screening for and/or diagnosis of renal cell cancer therapy, in a subject, which method comprises the step of detecting and/or quantifying in a biological sample obtained from said subject, a CD27L polypeptide which:
(a) comprises or consists of the amino acid sequence shown in Figure 1 (SEQ ID NO:2); wherein said step comprises the use of an antibody that specifically binds to the CD27L polypeptide.

In a further embodiment, the level of the CD27L polypeptide is compared to a reference range or control.

The polypeptides described in (a) above are hereinafter referred to as "CD27L polypeptides". The term "polypeptides" includes peptides, polypeptides and proteins. These are used interchangeably unless otherwise specified.

A CD27L nucleic acid molecule:
(d) comprises or consists of the DNA sequence shown in Figure 1 (SEQ ID NO: 1) or its RNA equivalent;
(e) has a sequence which is complementary to the sequences of (d);
(f) has a sequence which codes for a polypeptide as defined in (a) to (c) above;
(g) has a sequence which shows substantial identity with any of those of (d), (e) and (f); or
(h) is a fragment of (d), (e), (f) or (g), which is at least 8 nucleotides in length.

The nucleic acid molecules described in (d) to (h) above are hereinafter referred to as "CD27L nucleic acids".

The epithelial-derived cancer is kidney cancer, specifically the kidney cancer is renal cell cancer. The subject may be a mammal and is preferably a human.

In further aspect, the present invention provides the use of an antibody that specifically binds to CD27L in the manufacture of a composition for the treatment of renal cell cancer, said antibody being conjugated to a therapeutic agent or drug moiety.

In a further aspect, the present invention provides an antibody that specifically binds to CD27L for use in the treatment of renal cell cancer, said antibody being conjugated to a therapeutic agent or drug moiety.

In another aspect, the present invention provides a pharmaceutical composition comprising an antibody that specifically binds to CD27L for use in the treatment of renal cell cancer, said antibody being conjugated to a therapeutic agent or drug moiety.

In the aspects above, the polypeptides or fragments thereof may be provided in isolated or recombinant form, and may be fused to other moieties. The polypeptides or fragments thereof may be provided in substantially pure form, that is to say free, to a substantial extent, from other proteins. Thus, a polypeptide may be provided in a composition in which it is the predominant component present (i.e. it is present at a level of at least 50%; preferably at least 75%, at least 80%, at least 85%, at least 90%, at least 95% or at least 98%; when determined on a weight/weight basis excluding solvents or carriers).

In further aspects of the invention, the antibody that specifically binds to CD27L may be used as part of diagnostic assays including screening assays, to identify the presence or instances of renal cell cancer in a patient, , all as more fully described and illustrated herein. Further encompassed by the invention are methods for monitoring renal cell cancer treatment in a patient, comprising the step of quantifying the expression or activity of CD27L in a patient undergoing treatment for renal cell cancer.

Accordingly, the present invention will be better understood from a consideration of the ensuing detailed description which proceeds with reference to the following drawing figures.

### BRIEF DESCRIPTION OF THE FIGURES

**Figure 1****:** shows the nucleotide (SEQ ID NO:1) and amino acid (SEQ ID NO:2) sequences of CD27L.
The tandem mass spectra are in bold and underlined.
**Figure 2****:** shows tissue distribution of CD27L mRNA. Levels of mRNA in normal tissues and renal carcinoma cell lines were quantified by real time RT-PCR. mRNA levels are expressed as the number of copies ng⁻¹ cDNA.
**Figure 3****:** shows the expression of CD27L in normal and tumour colon tissues. Levels of CD27L mRNA in matched normal and tumour tissues from three patients were measured by real time RT-PCR. mRNA levels are expressed as the number of copies ng⁻¹ cDNA.
**Figure 4****:** shows the expression of CD27L in a number of kidney tumour tissues, including two pairs of matched normal and tumour tissues. Levels of CD27L mRNA were measured by real time RT-PCR. mRNA levels are expressed as the number of copies ng⁻¹ cDNA.

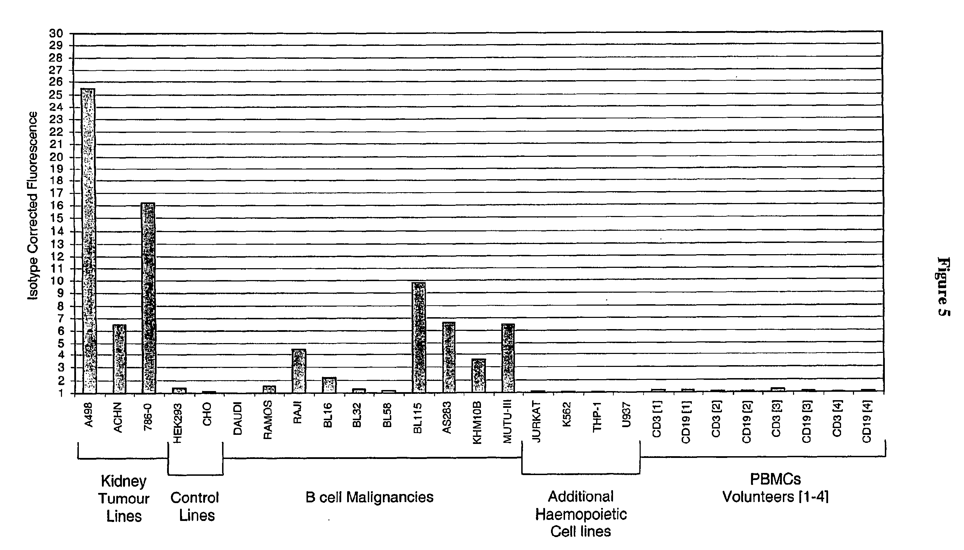

**Figure 5****:** shows the relative expression of CD27L estimated by flow cytometry using an FITC labelled anti-CD27L antibody

### DETAILED DESCRIPTION

In order to more fully appreciate the present invention, polypeptides within the scope of (a) above will now be discussed in greater detail.

### Polypeptides within the scope of (a):

A polypeptide within the scope of (a), may consist of the particular amino acid sequence given in Figure 1 (SEQ ID NO:2) or may have an additional N-terminal and/or an additional C-terminal amino acid sequence relative to the sequence given in Figure 1 (SEQ ID NO:2). Additional N-terminal or C-terminal sequences may be provided for various reasons. Techniques for providing such additional sequences are well known in the art. Additional sequences may be provided in order to alter the characteristics of a particular polypeptide. This can be useful in improving expression or regulation of expression in particular expression systems. For example, an additional sequence may provide some protection against proteolytic cleavage. This has been done for the hormone Somatostatin by fusing it at its N-terminus to part of the β galactosidase enzyme (Itakwa et al. (1977) Science 198: 105-63).

Additional sequences can also be useful in altering the properties of a polypeptide to aid in identification or purification. For example, a fusion protein may be provided in which a polypeptide is linked to a moiety capable of being isolated by affinity chromatography. The moiety may be an antigen or an epitope and the affinity column may comprise immobilised antibodies or immobilised antibody fragments which bind to said antigen or epitope (desirably with a high degree of specificity). The fusion protein can usually be eluted from the column by addition of an appropriate buffer. Additional N-terminal or C-terminal sequences may, however, be present simply as a result of a particular technique used to obtain a polypeptide of the present invention and need not provide any particular advantageous characteristic to the polypeptide of the present invention. Such polypeptide are within the scope of the present invention.

Whatever additional N-terminal or C-terminal sequence is present, it is preferred that the resultant polypeptide should exhibit the binding ability of a CD27L polypeptide as shown in Figure 1 (SEQ ID NO:2).

In order to more fully appreciate the present invention, nucleic acids within the scope of (d)-(h) above will now be discussed in greater detail.

CD27L polypeptides can be coded for by a large variety of nucleic acid molecules, taking into account the well known degeneracy of the genetic code. All of these molecules can be utilised in the present invention. They can be inserted into vectors and cloned to provide large amounts of DNA or RNA for further study. Suitable vectors may be introduced into host cells to enable the expression of polypeptides of the present invention using techniques known to the person skilled in the art.

Techniques for cloning, expressing and purifying polypeptides are well known to the skilled person. DNA constructs can readily be generated using methods well known in the art. These techniques are disclosed, for example in Sambrook et al, Molecular Cloning 2nd Edition, Cold Spring Harbour Laboratory Press (1989); in Old & Primrose Principles of Gene Manipulation 5th Edition, Blackwell Scientific Publications (1994); and in Stryer [Biochemistry 4th Edition, W H Freeman and Company (1995)]. Modifications of DNA constructs and the proteins expressed such as the addition of promoters, enhancers, signal sequences, leader sequences, translation start and stop signals and DNA stability controlling regions, or the addition of fusion partners may then be facilitated.

Normally the DNA construct will be inserted into a vector, which may be of phage or plasmid origin. Expression of the protein is achieved by the transformation or transfection of the vector into a host cell which may be of eukaryotic or prokaryotic origin. Such vectors and suitable host cells form third and fourth aspects of the present invention.

Knowledge of the nucleic acid structure can be used to raise antibodies and for gene therapy. Techniques for this are well-known by those skilled in the art.

By using appropriate expression systems, CD27L polypeptides may be expressed in glycosylated or non-glycosylated form. Non-glycosylated forms can be produced by expression in prokaryotic hosts, such as *E*. *coli*.

Polypeptides comprising N-terminal methionine may be produced using certain expression systems, while in others the mature polypeptide will lack this residue.

Preferred techniques for cloning, expressing and purifying a CD27L polypeptide are summarised below.

Polypeptides may be prepared natively or under denaturing conditions and then subsequently refolded. Baculoviral expression vectors include secretory plasmids (such as pACGP67 from Pharmingen), which may have an epitope tag sequence cloned in frame (e.g. myc, V5 or His) to aid detection and allow for subsequent purification of the protein. Mammalian expression vectors may include pCDNA3 and pSecTag (both Invitrogen), and pREP9 and pCEP4 (invitrogen). *E. coli* systems include the pBad series (His tagged - Invitrogen) or pGex series (Pharmacia).

The term "identity" can be used to describe the similarity between two individual DNA sequences. The 'bestfit' program (Smith and Waterman, Advances in Applied Mathematics, 482-489 (1981)) is one example of a type of computer software used to find the best segment of similarity between two nucleic acid sequences, whilst the GAP program enables sequences to be aligned along their whole length and finds the optimal alignment by inserting spaces in either sequence as appropriate. It is preferred if sequences which show substantial identity with any of those of (d), (e) or (f) have e.g. at least 50%, at least 75%, at least 80%, at least 85%, at least 90%, at least 95% or at least 98% sequence identity.

In addition to nucleic acid molecules coding for CD27L polypeptides, referred to herein as "coding" nucleic acid molecules, the present invention also includes nucleic acid molecules complementary thereto. Thus, for example, both strands of a double stranded nucleic acid molecule may be utilised in the present invention (whether or not they are associated with one another). Also included are mRNA molecules and complementary DNA Molecules (e.g. cDNA molecules).

Nucleic acid molecules which can hybridise to any of the nucleic acid molecules discussed above may also be utilised by the present invention. Such nucleic acid molecules are referred to herein as "hybridising" nucleic acid molecules. Hybridising nucleic acid molecules can be useful as probes or primers, for example. Desirably such hybridising molecules are at least 10 nucleotides in length and preferably are at least 25 or at least 50 nucleotides in length. The hybridising nucleic acid molecules preferably hybridise to nucleic acids within the scope of (d), (e), (f) or (g) above specifically. Desirably the hybridising molecules will hybridise to such molecules under stringent hybridisation conditions. One example of stringent hybridisation conditions is where attempted hybridisation is carried out at a temperature of from about 35°C to about 65°C using a salt solution which is about 0.9 molar. However, the skilled person will be able to vary such conditions as appropriate in order to take into account variables such as probe length, base composition, type of ions present, etc.

Manipulation of the DNA encoding the protein is a particularly powerful technique for both modifying proteins and for generating large quantities of protein for purification purposes. This may involve the use of PCR techniques to amplify a desired nucleic acid sequence. Thus the sequence data provided herein can be used to design primers for use in PCR so that a desired sequence can be targeted and then amplified to a high degree. Typically primers will be at least five nucleotides long and will generally be at least ten nucleotides long (e.g. fifteen to twenty-five nucleotides long). In some cases, primers of at least thirty or at least thirty-five nucleotides in length may be used.

As a further alternative, chemical synthesis may be used. This may be automated. Relatively short sequences may be chemically synthesised and ligated together to provide a longer sequence. In addition to being used as primers and/or probes, hybridising nucleic acid molecules of the present invention can be used as anti-sense molecules to alter the expression of CD27L polypeptides by binding to complementary nucleic acid molecules. This technique can be used in anti-sense therapy. A hybridising nucleic acid molecule of the present invention may have a high degree of sequence identity along its length with a nucleic acid molecule within the scope of(d)-(h) above (e.g. at least 50%, at least 75%, at least 80%, at least 85%, at least 90%, at least 95% or at least 98% sequence identity). As will be appreciated by the skilled person, the higher the sequence identity a given single stranded nucleic acid molecule has with another nucleic acid molecule, the greater the likelihood that it will hybridise to a nucleic acid molecule which is complementary to that other nucleic acid molecule under appropriate conditions.

The term 'RNA equivalent' when used above indicates that a given RNA molecule has a sequence which is complementary to that of a given DNA molecule, allowing for the fact that in RNA 'U' replaces 'T' in the genetic code. The nucleic acid molecule may be in isolated, recombinant or chemically synthetic form.

In view of the foregoing description the skilled person will appreciate that a large number of nucleic acids are within the scope of the present invention. Unless the context indicates otherwise, nucleic acid molecules of the present invention may additionally have one or more of the following characteristics:
- they may be single or double stranded;
- they may be provided in recombinant form i.e. covalently linked to a 5' and/or a 3' flanking sequence to provide a molecule which does not occur in nature;
- they may be provided without 5' and/or 3' flanking sequences which normally occur in nature; and
- they may be provided in substantially pure form. Thus they may be provided in a form which is substantially free from contaminating proteins and/or from other nucleic acids;

As described herein, CD27L is associated with epithelial-derived cancer, in particular kidney cancer and/or colorectal cancer, and as such provides a means of detection and/or diagnosis. In one embodiment, a convenient means for such detection, quantifying, screening, diagnosis, prophylaxis, or therapeutic treatment, will involve the use of antibodies. Thus, the CD27L polypeptides also find use in raising antibodies. Thus, in a further aspect, the present invention utilises antibodies, which bind to a CD27L polypeptide. Preferred antibodies bind specifically to CD27L polypeptides so that they can be used to purify and/or inhibit the activity of such polypeptides.

Thus, CD27L polypeptides may be used as immunogens to generate antibodies which immunospecifically bind a CD27L polypeptide. These are referred to herein as CD27L antibodies. CD27L antibodies include, but are not limited to polyclonal, monoclonal, bispecific, humanised or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically active portions of immunoglobulin molecules, i.e., molecules that contain an antigen binding site that specifically binds an antigen. The immunoglobulin molecules of the invention can be of any class (e.g., IgG, IgE, IgM, IgD and IgA) or subclass of immunoglobulin molecule.

In the production of antibodies, screening for the desired antibody can be accomplished by techniques known in the art, e.g. ELISA (enzyme-linked immunosorbent assay). For example, to select antibodies which recognise a specific domain of a CD27L polypeptide, one may assay generated hybridomas for a product which binds to a polypeptide fragment containing such domain. For selection of an antibody that specifically binds a first polypeptide homolog but which does not specifically bind to (or binds less avidly to) a second polypeptide homolog, one can select on the basis of positive binding to the first polypeptide homolog and a lack of binding to (or reduced binding to) the second polypeptide homolog.

For preparation of monoclonal CD27L antibodies (mAbs), any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo*. In an additional embodiment of the invention, mAbs can be produced in germ-free animals utilising known technology (PCT/US90/02545).

The mAbs include but are not limited to human mAbs and chimeric mAbs (e.g., human-mouse chimeras). A chimeric antibody is a molecule in which different portions are derived from different animal species, such as those having a human immunoglobulin constant region and a variable region derived from a murine mAb. (See, U.S. 4,816,567; and U.S. 4,816397) Humanised antibodies are antibody molecules from non-human species having one or more complementarity determining regions (CDRs) from the non-human species and a framework region from a human immunoglobulin molecule. (See, e.g., U.S. 5,585,089)

Chimeric and humanised mAbs can be produced by recombinant DNA techniques known in the art, for example using methods described in WO 87/02671; EP 184,187; EP 171,496; EP 173,494; WO 86/01533; U.S. 4,816,567; EP 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Canc. Res. 47:999-1005; Wood et al. (1985) Nature 314:446-449; and Shaw et al. (1988) J. Natl. Cancer Inst. 80:1553-1559; Morrison (1985) Science 229:1202-1207; Oi et al. (1986,)Bio/Techniques 4:214; U.S. Patent 5,225,539; Jones et al. (1986) Nature 321:552-525; Verhoeyan et al. (1988) Science 239:1534; and Beidler et al. (1988) J. Immunol. 141:4053-4060.

Completely human antibodies are particularly desirable for therapeutic treatment of human patients. Such antibodies can be produced using transgenic mice which are incapable of expressing endogenous immunoglobulin heavy arid light chain genes, but which can express human heavy and light chain genes. The transgenic mice are immunised in the normal fashion with a selected antigen, e.g., all or a portion of a CD27L polypeptide. MAbs directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human mAbs and protocols for producing such antibodies, see, e.g., U.S. Patent 5,625,126; U.S. Patent 5,633,425; U.S. Patent 5,569,825; U.S. Patent 5,661,016; and U.S. Patent 5,545,806.

Completely human antibodies which recognise a selected epitope can be generated using a technique referred to as "guided selection." In this approach a selected non-human monoclonal antibody, e.g., a mouse antibody, is used to guide the selection of a completely human antibody recognising the same epitope. (Jespers et al. (1994) Bio/technology 12:899-903).

The CD27L antibodies can also be generated using various phage display methods known in the art. In phage display methods, functional antibody domains are displayed on the surface of phage particles which carry the polynucleotide sequences encoding them. In a particular, such phage can be utilised to display antigen binding domains expressed from a repertoire or combinatorial antibody library (e.g., human or murine). Phage expressing an antigen binding domain that binds the antigen of interest can be selected or identified with antigen, e.g., using labelled antigen or antigen bound or captured to a solid surface or bead. Phage used in these methods are typically filamentous phage including fd and M13 binding domains expressed from phage with Fab, Fv or disulfide stabilised Fv antibody domains recombinantly fused to either the phage gene III or gene VIII protein. Phage display methods that can be used to make the antibodies of the present invention include those disclosed in Brinkman et al (1995) J. Immunol. Methods 182:41-50; Ames et al. (1995) J. Immunol. Methods 184:177-186; Kettleborough et al. (1994) Eur. J. Immunol. 24:952-958; Persic et al. (1997) Gene 187 9-18; Burton et al. (1994) Advances in Immunology 57:191-280; PCT Application No. PCT/GB91/01134; WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108;.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in WO 92/22324; Mullinax et al. (1992) BioTechniques 12(6):864-869; and Sawai et al. (1995) AJRI 34:26-34 and Better et al. (1988) Science 240:1041-1043.

Examples of techniques which can be used to produce single-chain Fvs and antibodies include those described in U.S. Patents 4,946,778 and 5,258,498; Huston et al. (1991) Methods in Enzymology 203:46-88Shu et al. (1993) PNAS 90:7995-7999; and Skerra et al. (1988) Science 240:1038-1040.

The invention further provides for the use of bispecific antibodies, which can be made by methods known in the art. Traditional production of full length bispecific antibodies is based on the co-expression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In one embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details for generating bispecific antibodies see, for example, Suresh et al., Methods in Enzymology, 1986, 121:210.

The invention also utilises functionally active fragments, derivatives or analogs of the CD27L antibodies. Functionally active means that the fragment, derivative or analog is able to elicit anti-anti-idiotype antibodies (i.e., tertiary antibodies) that recognise the same antigen that is recognised by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognises the antigen. To determine which CDR sequences bind the antigen, synthetic peptides containing the CDR sequences can be used in binding assays with the antigen by any binding assay method known in the art.

The present invention utilises antibody fragments such as, but not limited to, F(ab')2 fragments and Fab fragments. Antibody fragments which recognise specific epitopes may be generated by known techniques. F(ab')2 fragments consist of the variable region, the light chain constant region and the CH1 domain of the heavy chain and are generated by pepsin digestion of the antibody molecule. Fab fragments are generated by reducing the disulfide bridges of the F(ab')2 fragments. The invention also provides heavy chain and light chain dimmers of the antibodies of the invention, or any minimal fragment thereof such as Fvs or single chain antibodies (SCAs) (e.g., as described in U.S. Patent 4,946,778; Bird (1988) Science 242:423-42; Huston et al. (1988) Proc. Natl. Acad. Sci. USA 85:5879-5883; and Ward et al. (1989) Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in E. coli may be used (Skerra et al. (1988) Science 242:1038-1041).

In other embodiments, the invention utilises fusion proteins of the CD27L antibodies (or functionally active fragments thereof), for example in which the immunoglobulin is fused via a covalent bond (e.g., a peptide bond), at either the N-terminus or the C-terminus to an amino acid sequence of another protein (or portion thereof, preferably at least 10, 20 or 50 amino acid portion of the protein) that is not the immunoglobulin. Preferably the CD27L antibody, or fragment thereof, is covalently linked to the other protein at the N-terminus of the constant domain. As stated above, such fusion proteins may facilitate purification, increase half-life in vivo, and enhance the delivery of an antigen across an epithelial barrier to the immune system.

The CD27L antibodies utilised include analogs and derivatives that are either modified, i.e., by the covalent attachment of any type of molecule as long as such covalent attachment that does not impair immunospecific binding. For example, but not by way of limitation, the derivatives and analogs of the CD27L antibodies include those that have been further modified, e.g., by glycosylation, acetylation, pegylation, phosphylation, amidation, derivatisation by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical amino acids.

The foregoing CD27L antibodies can be used in methods known in the art relating to the localisation and activity of the CD27L polypeptides, e.g., for imaging or radioimaging these proteins, measuring levels thereof in appropriate physiological samples, in diagnostic methods, etc. and for radiotherapy.

The CD27L antibodies can be produced by any method known in the art for the synthesis of antibodies, in particular, by chemical synthesis or by recombinant expression, and are preferably produced by recombinant expression technique.

Recombinant expression of CD27L antibodies, requires construction of a nucleic acid that encodes the antibody. If the nucleotide sequence of the antibody is known, a nucleic acid encoding the antibody may be assembled from chemically synthesised oligonucleotides (e.g., as described in Kutmeier et al. (1994) BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g., an antibody cDNA library, or cDNA library generated from any tissue or cells expressing the antibody) by PCR amplification using synthetic primers hybridisable to the 3' and 5' ends of the sequence or by cloning using an oligonucleotide probe specific for the particular gene sequence.

If an antibody molecule that specifically recognises a particular antigen is not available (or a source for a cDNA library for cloning a nucleic acid encoding such an antibody), antibodies specific for a particular antigen may be generated by any method known in the art, for example, by immunising an animal, such as a rabbit, to generate polyclonal antibodies or, more preferably, by generating monoclonal antibodies. Alternatively, a clone encoding at least the Fab portion of the antibody may be obtained by screening Fab expression libraries (e.g., as described in Huse et al., 1989, Science 246:1275-1281) for clones ofFab fragments that bind the specific antigen or by screening antibody libraries (See, e.g., Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, e.g., WO 86/05807; WO 89/01036; and U.S. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, in vitro site directed mutagenesis (Hutchinson et al., 1978, J. Biol. Chem. 253:6551), PCR based methods, etc.

In addition, techniques developed for the production of "chimeric antibodies" (Morrison et al. (1984) Proc. Natl. Acad. Sci. 81:851-855; Neuberger et al. (1984) Nature 312:604-608; Takeda et al. (1985) Nature 314:452-454) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human antibody constant region, e.g., humanised antibodies.

Once a nucleic acid encoding a CD27L antibody has been obtained, the vector for the production of the antibody molecule may be produced by recombinant DNA technology using techniques well known in the art. Thus, methods for preparing the protein of the invention by expressing nucleic acid containing the antibody molecule sequences are described herein. Methods which are well known to those skilled in the art can be used to construct expression vectors containing an antibody molecule coding sequences and appropriate transcriptional and translational control signals. These methods include, for example, in vitro recombinant DNA techniques, synthetic techniques, and in vivo genetic recombination. See, for example, the techniques described in Sambrook et al. (1990), Molecular Cloning, A Laboratory Manual, 2d Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant CD27L antibody may be either bacterial cells such as *Escherichia coli,* or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking et al. (1998) Gene 45:101; Cockett et al. (1990) Bio/Technology 8:2).

A variety of host-expression vector systems may be utilised to express a CD27L antibody. Such host-expression systems represent vehicles by which the coding sequences of interest may be produced and subsequently purified, but also represent cells which may, when transformed or transfected with the appropriate nucleotide coding sequences, express the antibody molecule of the invention in situ. These include but are not limited to microorganisms such as bacteria (e.g., *E. coli, B. subtilis*) transformed with recombinant bacteriophage DNA, plasmid DNA or cosmid DNA expression vectors containing antibody coding sequences; yeast (e.g., *Saccharomyces, Pichia*) transformed with recombinant yeast expression vectors containing antibody coding sequences; insect cell systems infected with recombinant virus expression vectors (e.g., baculovirus) containing the antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, HEK 293, 3T3 cells) harbouring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the E. coli expression vector pUR278 (Ruther et al., 1983, EMBO J. 2:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (e.g., an adenovirus expression system) may be utilised.

As discussed above, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein.

For long-term, high-yield production of recombinant CD27L antibodies, stable expression is preferred. For example, cells lines that stably express a CD27L antibody can be produced by transfecting the cells with an expression vector comprising the nucleotide sequence of the antibody and the nucleotide sequence of a selectable (e.g., neomycin or hygromycin), and selecting for expression of the selectable marker. Such engineered cell lines may be particularly useful in screening and evaluation of agents that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the CD27L antibody molecule has been recombinantly expressed, it may be purified by any method known in the art for purification of an antibody molecule, for example, by chromatography (e.g., ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilising an antibody specific for the fusion protein being expressed. For example, a system described by Janknecht *et al.* allows for the ready purification of non-denatured fusion proteins expressed in human cell lines (Janknecht et al. (1991 Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

In a preferred embodiment, CD27L antibodies or fragments thereof are conjugated to a diagnostic moiety. The CD27L antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and 99 Tc.

CD27L antibodies for use in the treatment of renal cell cancer are conjugated to a therapeutic agent or drug moiety. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

Alternatively, an antibody can be conjugated to a second antibody to form an antibody heteroconjugate as described in U.S. Patent No. 4,676,980.

An antibody with a therapeutic moiety conjugated to it can be used as a therapeutic that is administered alone or in combination with cytotoxic factor(s) and/or cytokine(s).

The methods of diagnosis according to the present invention may be performed using a number of methods known to those skilled in the art, including, without limitation, immunoprecipitation followed by sodium dodecyl sulfate polyacrylamide gel electrophoresis, 2 dimensional gel electrophoresis, immunocytochemistry, immunohistochemistry, immunoassays, e.g. western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

Diagnostic kits may comprise a capture reagent (e.g. an antibody) against a CD27L polypeptide as defined above. In addition, such a kit may optionally comprise one or more of the following:
(1) instructions for using the capture reagent for diagnosis, prognosis, therapeutic monitoring or any combination of these applications;
(2) a labelled binding partner to the capture reagent;
(3) a solid phase (such as a reagent strip) upon which the capture reagent is immobilised; and
(4) a label or insert indicating regulatory approval for diagnostic, prognostic or therapeutic use or any combination thereof.

If no labelled binding partner to the capture reagent is provided, the anti-polypeptide capture reagent itself can be labelled with a detectable marker, e.g. a chemiluminescent, enzymatic, fluorescent, or radioactive moiety (see above).

A diagnostic kit comprises in one or more containers a pair of primers that under appropriate reaction conditions can prime amplification of at least a portion of a CD27L nucleic acid molecule, such as by polymerase chain reaction (see e.g., Innis et al., 1990, PCR Protocols, Academic Press, Inc., San Diego, CA), ligase chain reaction (see EP 320,308) use of Qβ replicase, cyclic probe reaction, or other methods known in the art. Typically, primers are at least five nucleotides long and will preferably be at least ten to twenty-five nucleotides long and more preferably fifteen to twenty-five nucleotides long. In some cases, primers of at least thirty or at least thirty-five nucleotides in length may be used.

The biological sample can be obtained from any source, such as a serum sample or a tissue sample, e.g. kidney or colon tissue. When looking for evidence of metastasis, one would look at major sites of kidney cancer metastasis, such as lymph nodes, liver, and pancreas.

As discussed herein, the CD27L antibodies find use in the treatment or prophylaxis of renal cell cancer. Thus, in another aspect, the present invention provides a pharmaceutical composition comprising at least one CD27L antibody conjugated to a therapeutic agent or drug moiety, optionally together with one or more pharmaceutically acceptable excipients, carriers or diluents for use in the diagnosis, treatment and/or prophylaxis of renal cell cancer.

In one aspect, the present invention provides a CD27L antibody conjugated to a therapeutic agent or drug moiety for use in the treatment of renal cell cancer. In another aspect, the present invention provides the use of at least one CD27L antibody conjugated to a therapeutic agent or drug moiety in the preparation of a composition for use in the diagnosis, prophylaxis and/or treatment of renal cell cancer.

The composition will usually be supplied as part of a sterile, pharmaceutical composition which will normally include a pharmaceutically acceptable carrier. This pharmaceutical composition may be in any suitable form, (depending upon the desired method of administering it to a patient). It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc. For the preparation of solutions and syrups, excipients which may be used include for example water, polyols and sugars. For the preparation of suspensions, oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research (1986) 3(6):318.

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulisers or insulators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilised) condition requiring only the addition of the sterile liquid carried, for example water for injections, immediately prior to use. Extemporaneous injection solutions and suspensions may be prepared from sterile powders, granules and tablets.

Sterile injectable solutions can be prepared by incorporating the agent in the required amount in an appropriate solvent with one or a combination of ingredients enumerated above, as required, followed by sterilization microfiltration.

In certain embodiments, the agents of the invention can be formulated to ensure proper distribution in vivo, for example, in liposomes. For methods of manufacturing liposomes, see, e. g., U. S. Patents 4,522,811; 5,374,548; and 5,399,331. The liposomes may comprise one or more moieties which are selectively transported into specific cells or organs, thus enhance targeted drug delivery (see, e. g., V. V. Ranade (1989) J. Clin. Pharmacol. 29: 685).

Exemplary targeting moieties include folate or biotin (see, e. g., U. S. Patent 5,416,016); mannosides (Umezawa et al., (1988) Biochem. Biophys. Res. Commun. 153: 1038); antibodies (P. G. Bloeman et al. (1995) FEBSLett. 357: 140; M. Owais et al. (1995) Antimicrob. Agents Chemother. 39: 180); surfactant protein A receptor (Briscoe et al. (1995) Am. J. Physiol. 1233: 134), different species of which may comprise the formulations of the inventions, as well as components of the invented molecules; psi 20 (Schreier et al. (1994) J. Biol. Chem. 269: 9090); see also K. Keinanen; M. L. Laukkanen (1994) FEBSLett. 346: 123; J. J. Killion; I. J. Fidler (1994) Immunomethods 4 : 273. In one embodiment of the invention, the agents of the invention are formulated in liposomes; in a more preferred embodiment, the liposomes include a targeting moiety. In a most preferred embodiment, the agents in the liposomes are delivered by bolus injection to a site proximal to the tumor.

The composition must be fluid to the extent that easy syringability exists. It must be stable under the conditions of manufacture and storage and must be preserved against the contaminating action of microorganisms such as bacteria and fungi.

The composition must be sterile and fluid to the extent that the composition is deliverable by syringe. In addition to water, the carrier can be an isotonic buffered saline solution, ethanol, polyol (for example, glycerol, propylene glycol, and liquid polyetheylene glycol, and the like), and suitable mixtures thereof. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as manitol or sorbitol, and sodium chloride in the composition. Long-term absorption of the injectable compositions can be brought about by including in the composition an agent which delays absorption, for example, aluminum monostearate or gelatin.

The pharmaceutical compositions may contain preserving agents, solubilising agents, stabilising agents, wetting agents, emulsifiers, sweeteners, colourants, odourants, salts (agents of the present invention may themselves be provided in the form of a pharmaceutically acceptable salt), buffers, coating agents or antioxidants. They may also contain therapeutically active agents in addition to the agent of the present invention.

Dosages of the agent of the present invention can vary between wide limits, depending upon the disease or disorder to be treated, the age and condition of the individual to be treated, etc. and a physician will ultimately determine appropriate dosages to be used. This dosage may be repeated as often as appropriate. If side effects develop the amount and/or frequency of the dosage can be reduced, in accordance with normal clinical practice.

The following examples are presented in order to more fully illustrate the preferred embodiments of the invention. They should in no way be construed, however, as limiting the scope of the invention.

### EXAMPLES

### Example 1: Identification and cloning of CD27L

Protein CD27L was isolated from A 498 cell membranes. A 498 is a renal epithelial carcinoma cell (ATCC no CRL-7908). This cell was grown in DMEM medium containing 10% fetal bovine serum and 2 mM L-glutamine.

### Cell fractionation and plasma membrane generation:

10 x 15cm² dishes of cells were washed three times with PBS-CM (each dish contained approx. 2x10e8 cells). 5 mls of ice cold PBS-CM was added to the first dish and the cells were scraped using a plastic cell lifter. All the dishes were scraped in this volume of PBS-CM. The cells were then centrifuged at 1000 x g for 5 minutes at +4°C. The supernatant was removed and the cells were resuspended in 10 mls of homogenizing buffer (250 mM sucrose in 10 mM HEPES, 1 mM EDTA 1 mM Vanadate, 0.02% Azide). The cells were centrifuged at 1000 x g for 5 minutes at +4°C and the supernatant removed. The cell pellet was then resuspended in 5 x packed cell volume with homogenizing buffer plus protease inhibitors (Sigma). The ball bearing homogeniser (BBH) (8.002mm ball) was chilled and rinsed with homogenizing buffer. The cell suspension was taken up in a 2 ml syringe and this was attached to one side of the BBH. Another syringe was attached to the other side of the BBH. The cell mixture was fed through the chamber up to five times. The cells were monitored using a microscope and when the cells were sufficiently lysed the resulting mixture was centrifuged at 1000 x g for 5 minutes at +4°. The resulting supernatant (PNS) was retained. 1 ml of homogenizing buffer was added to the nuclear pellet and re-centrifuged at 1000 x g for 5 minutes. The above two fractions were pooled and centrifuge at 3000 x g for 10 minutes at +4°C. The 3000 x g supernatant was layered onto 2 ml 60% sucrose cushion in SW40 or SW60 tube and centrifuged at 100,000 x g for 45 minutes with slow acceleration and deceleration. The crude plasma membrane was evident as a discrete layer on top of the sucrose cushion. The upper layer was removed (cytosol) and the plasma membrane was collected using a pasteur pipette. The % sucrose of crude plasma membrane fraction was determined using a refractometer. The membrane preparation was diluted with HEPES buffer to reduce the sucrose content to below 15%. The crude plasma membrane preparation was layered on preformed 15 to 60% sucrose gradient in SW40 tube and spun at 100 000 x g for 17 hours with slow acceleration and deceleration. The sucrose gradient was fractionated using the gradient unloader (speed 0.5, distance 2.5, fractions 35). The protein content of the fractions was measured and 10 micrograms of protein was run on a 4-20% acrylamide 1D gel (Novex) and subject to western blotting with antibodies to Transferrin Receptor, Oxidoreductase II and Calnexin.

### Preparation of plasma membrane fractions for 1D gel analysis:

The plasma membrane fractions were identified which had transferrin immunoreactivity but no oxidoreductase II or calnexin immunoreactivity. The sucrose fractions were pooled and diluted at least four times with 10 mM HEPES, 1 mM EDTA 1 mM vanadate, 0.02% azide. The diluted sucrose fraction was added to a SW40 or SW60 tube and centrifuged at 100,000 x g for 45 minutes with slow acceleration and deceleration. The supernatant was removed from the membrane pellet and the pellet was washed three times with PBS-CM. The membrane pellet was solubilized in 2% SDS in 63 mM TrisHCL pH 7.4. A protein assay was done and mercaptoethanol (2% final), glycerol (10%) and bromopheneol blue (0.0025% final) was added. A final protein concentration of 1 microgram/microlitre was used for 1D gel loading. The extracted protein sample was solubilized in 1D lysis buffer and the proteins separated by 1D PAGE (8 - 16% gradient).

### Mass Spectrometry

CD27L protein excised from the 1D gel was digested with trypsin and analyzed by MALDI-TOF-MS (Voyager STR, Applied Biosystems) using a 337 nm wavelength laser for desorption and the reflectron mode of analysis. Selected masses for CD27L ([M+H] = 1217.6 and 1695.8) were further characterised by tandem mass spectrometry using a QTOF-MS equipped with a nano liquid chromatography system eluting directly into the instrument. Prior to MALDI analysis the samples were desalted and concentrated using C18 Zip Tips™ (Millipore).

For partial amino acid sequencing and identification of CD27L, uninterpreted tandem mass spectra of tryptic peptides were searched using the SEQUEST search program (Eng et al., 1994, J. Am. Soc. Mass Spectrom. 5:976-989), version v.C.1.. The database searched was a database constructed of protein entries in the non-redundant database held by the National Centre for Biotechnology Information (NCB1) which is accessible at http://www.ncbi.nlm.nih.gov/. The tandem amino acid sequences were found to match the protein CD27L (available under the accession number P32970 in the SwissProt database, held by the Swiss Institute of Bioinformatics, (http://www.expasy.ch/)) (Figure 1). The predicted mass of the protein is 21.1 kDa, and the apparent molecular weight as measured on the gel is 23.7 kDa.

### Example 2: Expression of CD27L mRNA in human tissues

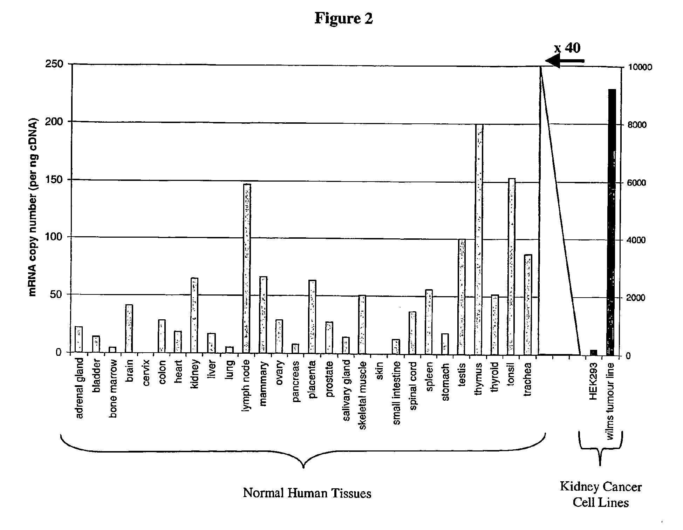

We used real time quantitative RT-PCR (Heid et al. (1996) Genome Res. 6, 986-994; Morrison et al. (1998) Biotechniques 24: 954-958) to analyse the distribution of CD27L mRNA in normal human tissues, kidney cancer cell lines and kidney and colon cancer clinical tissues (Fig 2-4).

### Preparation of total RNA and First Strand cDNA Synthesis.

Total RNA was prepared from cultured cells and homogenised tissue samples using Trizol reagent (Life Technologies), according to the manufacturer's instructions, and resuspended in RNAse-free water at a concentration of 1 µg/µl. First strand cDNA was synthesised from 5 µg of total RNA using Superscript II reverse transcriptase (Life Technologies), according to the manufacturer's instructions. Each cDNA sample was purified using a PCR purification mini-column (Qiagen), quantified by spectrophotometry at 260 nm, and diluted to 10 ng/µl.

### Quantification of CD27L mRNA by RT-PCR

Real time RT-PCR was used to quantitatively measure CD27L expression in normal human tissue mRNAs (Clontech), kidney cancer cell lines, and kidney and colon cancer tissues. The primers used for PCR were as follows:
sense, 5' gctgctttggtcccattggtcg 3' (SEQ ID NO:3)
antisense, 5' gaggtcctgtgtgattcagctg 3' (SEQ ID NO:4)

Reactions containing 10ng cDNA, prepared as described above, SYBR green sequence detection reagents (PE Biosystems) and sense and antisense primers were assayed on an ABI7700 sequence detection system (PE Biosystems). The PCR conditions were 1 cycle at 50°C for 2 min, 1 cycle at 95°C for 10 min, and 40 cycles of 95°C for 15s, 65°C for 1min. The accumulation of PCR product was measured in real time as the increase in SYBR green fluorescence, and the data were analysed using the Sequence Detector program vl.6.3 (PE Biosystems). Standard curves relating initial template copy number to fluorescence and amplification cycle were generated using the amplified PCR product as a template, and were used to calculate CD27L copy number in each sample.

As shown in Figure 2, the distribution of CD27L mRNA was low in normal tissues, with the highest levels of expression in thymus, tonsil and lymph node (147-200 copies ng⁻¹ cDNA), and only low levels of CD27L message detected in other normal tissues. CD27L mRNA was detected in the HEK 293 kidney cancer cell line (190 copies ng⁻¹ cDNA) and was highly overexpressed in Wilm's tumour cell line (kidney cancer in children, see *supra*) (9200 copies ng⁻¹ cDNA).

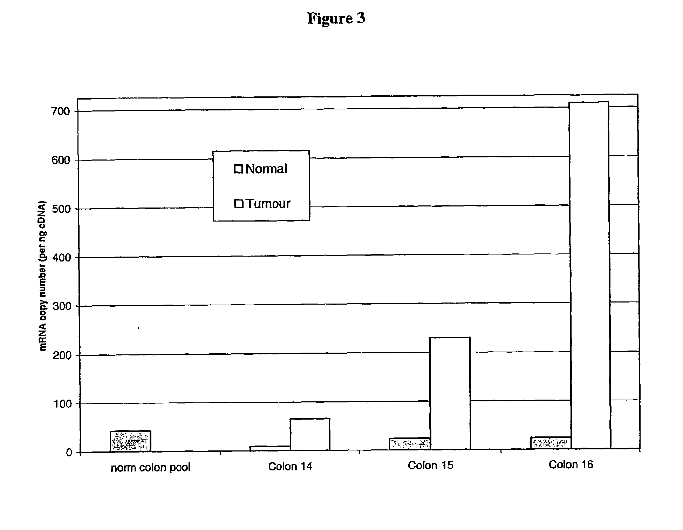

Figure 3 shows the levels of expression of CD27L in 3 matched normal/tumour colon pairs, along with the expression in normal colon. The levels of expression are increased in all sample pairs, between 7 and 30 times.

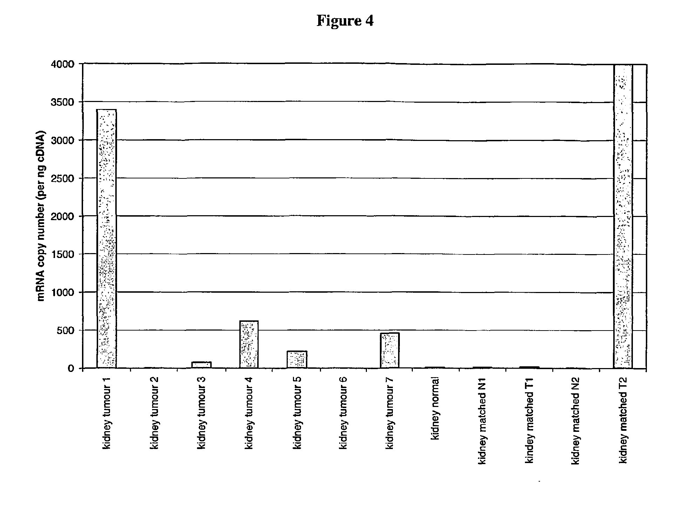

Figure 4 shows the level of expression of CD27L in seven kidney tumours, as well as in normal kidney tissues and in two matched pairs of tumour and normal kidney tissues. Elevated expression is observed in the majority of tumour samples, with levels in two samples above 3000 copies ng⁻¹ cDNA.

### Example 3 - The relative expression of CD27L was estimated by flow cytometry using an FITC labelled anti-CD27L antibody

Directly conjugated anti-CD27L antibody was obtained from Ancell Corp (Bayport, USA). This IgG1 antibody (designated BU69) was derived via immunisation of mice with the WM-1 (Waldenström's macroglobulinemia) cell line. The binding epitope has not been identified, however this antibody has been shown to inhibit T cell proliferation induced by dendritic cells and can therefore be viewed as 'neutralising', (see Leukocyte Typing V, S.F. Schlossman, et al, eds. Oxford University Press, Oxford, (1995) p. 1137-1138). The isotope control used was an FITC labelled IgGk1 antibody obtained from Serotec Ltd (Kidlington, UK). The following B cell lines: BL16, BL32, BL58, BL115, AS283 and KHM10B were provided by Prof. Martin Dyer (University of Leicester) whereas the MUTU-III line was provided by Dr. Noemi Nagy (Karolinska Institute, Sweden). All other lines were purchased from the ECACC or ATCC.

Adherent cell lines were harvested using standard non-enzymatic dissociation solution (Sigma). Suspension cells were harvested by removal of an appropriate amount of culture media from the flask. For peripheral blood mononuclear cell (PBMC) preparation; buffy coat preparations were obtained from the national blood service (NBS - John Radcliffe branch, Oxford, UK) under the terms of agreement 0211/T464. Pure cell suspensions were subsequently purified by centrifugation over Ficoll gradients (Sigma) according to the manufacturers instructions. Platelets were then depleted from PBMC cell suspensions by 4 rounds of low-speed centrifugation. All cell lines (adherent, suspension and primary volunteer PBMCs) were then washed a further time in DPBS, counted using a haemocytometer and resuspended at 5 million cells ml⁻¹.

### Flow cytometry protocol

For all cells to be analysed, a 100µl aliquot of cells (5x10⁵ cells) was added to each well on a 96 well plate. The cells were then pelleted by centrifugation at 2 krpm for 5 minutes and each pellet resuspended in 40µl of DPBS supplemented with 0.5%BSA. The plate was then incubated at 4 degrees for 20 minutes to allow blocking of hydrophobic binding sites on the cell surface. After incubation, 10µl of anti-CD27L antibody was added which had been previously diluted 1/10 from the original stock using DPBS [originally 1 µl per test, here using 10µl per test]. For all cells an additional sample was prepared in parallel, where the anti-CD27L antibody was substituted for a 10µl aliquot of IgG1k FITC isotype control antibody (Serotec Ltd.). The antibody labelled cells were then incubated for 1 hour at room temperature, with intermittent mixing (every 15 minutes). After incubation, cell were washed 3 times in standard DPBS and placed into polystyrene FACS tubes [Elkay Ltd.]. All flow cytometry acquisition was performed using an EPICS XL flow cytometer [Coulter Corp]. Cellular fluorescence was detected in FL1, cell aggregates were removed from the analysis by FSC/SSC gating. For data analysis, the [log] mean fluorescence intensity of the isotype control sample was subtracted from the [log] mean fluorescence intensities for the same cell line. This value, termed 'isotype corrected fluorescence' was then plotted for each cell line or peripheral blood sample and displayed in the form of a bar-chart. Standard errors not exceeding +/- 10% were viewed as acceptable for these experiments.

### Results

All kidney cell lines tested were found to express a significant level of CD27L. For two cell lines (A498 and 786-0) this fluorescence was the highest seen in the entire panel of cell lines. Amongst the B cell lines tested there was considerable variability in the levels of CD27L detected, with some cells not expressing at all. The human embryonic kidney line HEK-293 was also shown to be negative, as was the CHO cell line, included as an negative control. A T cell derived cell line (Jurkat), a myeloid leukemia line (K562) and two monocytic cell lines (U937 and THP-1) were also found to be negative for CD27L. With respect to peripheral blood subsets, for all 4 volunteers, both the CD3+ [T cell] and CD19+ (B cell) subsets were found to be CD27L negative.

Therefore, kidney cell lines have considerably higher CD27L expression that peripheral blood T and B cells. CD27L expression can be detected on a high proportion of B cell malignancies but not on myeloid lines. These findings suggest that a potential therapeutic antibody delivered into the circulation will not be sequestered by B/T cells expressing CD27L. Therefore, CD27L expression is most probably restricted to activated cells within lymphoid tissue i.e. Lymph node, Spleen, Tonsils etc.

### Example 4 - Summary: Binding of an anti- CD27L antibody to a panel of kidney tumour cell lines

Directly conjugated anti-CD27L antibody was obtained from Ancell Corp (Bayport, USA) as described above.

Cell lines were harvested by trypsin digestion and re-seeded onto chamber slides. After 24 hours incubation the chambers were washed three time in PBS and resuspended in 5% Formaldehyde to fix. After 5 minutes fixation cells were washed a further 3 times in DPBS and blocked in DPBS supplemented with 5% FBS. The cells were allowed to block for 60 minutes to prevent non-specific binding of antibody to cells. Primary FITC conjugated antibody (either anti-CD27L or IgG1 I isotype control) was then added (1 µg/ml in a 200µl 5% FBS DPBS) and the chamber slides incubated overnight at 4°C. Following incubation the cells were washed twice in DPBS/5% and once with DPBS alone, leaving at least 5 minutes between each wash step. The slides were then mounted in aqueous mounting media and visualised using a DMRIE2 fluorescence microscope (Leica AG).

For all 4 kidney tumour lines tested there was some element of reactivity with the anti-CD27L FITC antibody. The strongest CD27L staining was observed on A489 cells. Both control lines (CHO and HEK-293 cells) failed to show any specific fluorescence when incubated with anti-CD27L. A negligible signal was observed after incubation of all cell lines with the IgG1k-FITC control antibody.

Therefore, CD27L expression can be detected on all of the kidney tumour lines tested but was not found on the embryonic kidney line HEK-293 nor on the control CHO line.

## Claims

1. A method of screening for and/or diagnosis of renal cell cancer in a subject, and/or monitoring the effectiveness of renal cell cancer therapy, which comprises the step of detecting and/or quantifying in a biological sample obtained from said subject, a CD27L polypeptide which comprises or consists of the amino acid sequence shown in Figure 1 (SEQ ID NO: 2); wherein said step comprises the use of an antibody that specifically binds to the CD27L polypeptide.

2. The method of claim 1, wherein the level of said polypeptide is compared to a previously determined reference range or control.

3. The method according to claim 1 or 2, wherein the step of detecting comprises:
(a) contacting the sample with an antibody as defined in claim 1; and
(b) detecting whether binding has occurred between the antibody and said polypeptide in the sample.

4. The method according to claim 3, wherein step (b) comprises detecting the captured polypeptide using a directly or indirectly labelled detection reagent.

5. The method according to claim 3 or 4, wherein the antibody is immobilised on a solid phase.

6. The use of an antibody that specifically binds to CD27L in the manufacture of a composition for the treatment of renal cell cancer, said antibody being conjugated to a therapeutic agent or drug moiety.

7. An antibody that specifically binds to CD27L for use in the treatment of renal cell cancer, said antibody being conjugated to a therapeutic agent or drug moiety.

8. The use as claimed in claim 6 or the antibody as claimed in claim 7, wherein the drug moiety is a toxin.

9. The method according to claim 1 or the use according to claim 6 or the antibody as claimed in claim 7, wherein the antibody is polyclonal, monoclonal, chimeric, humanised or bispecific, or an epitope-binding fragment of any one thereof.

## Patentansprüche

1. Verfahren zur Suche nach und/oder Diagnose von Nierenzellkrebs bei einem Patienten und/oder zur Überwachung der Wirksamkeit einer Nierenzellkrebstherapie, wobei man in einem Verfahrensschritt in einer von dem Patienten erhaltenen biologischen Probe ein CD27L-Polypeptid, das die in Figur 1 (SEQ ID NO: 2) dargestellte Aminosäuresequenz umfasst oder daraus besteht, nachweist und/oder quantifiziert, wobei der Schritt die Verwendung eines Antikörpers umfasst, der spezifisch an das CD27L-Polypeptid bindet.

2. Verfahren nach Anspruch 1, wobei der Spiegel des Polypeptids mit einem bzw. einer zuvor bestimmten Referenzbereich bzw. -kontrolle verglichen wird.

3. Verfahren gemäß Anspruch 1 oder 2, wobei man in dem Nachweisschritt
(a) die Probe mit einem Antikörper mit der in Anspruch 1 angegebenen Bedeutung in Kontakt bringt und
(b) nachweist, ob eine Bindung zwischen dem Antikörper und dem Polypeptid in der Probe stattgefunden hat.

4. Verfahren gemäß Anspruch 3, wobei man in Schritt (b) das eingefangene Polypeptid unter Verwendung eines direkt oder indirekt markierten Nachweisreagens nachweist.

5. Verfahren gemäß Anspruch 3 oder 4, wobei der Antikörper an einer Festphase immobilisiert ist.

6. Verwendung eines Antikörpers, der spezifisch an CD27L bindet, bei der Herstellung einer Zusammensetzung zur Behandlung von Nierenzellkrebs, wobei der Antikörper an ein Therapeutikum oder eine Arzneistoffgruppierung konjugiert ist.

7. Antikörper, der spezifisch an CD27L bindet, zur Verwendung bei der Behandlung von Nierenzellkrebs, wobei der Antikörper an ein Therapeutikum oder eine Arzneistoffgruppierung konjugiert ist.

8. Verwendung nach Anspruch 6 oder Antikörper nach Anspruch 7, wobei es sich bei der Arzneistoffgruppierung um ein Toxin handelt.

9. Verfahren gemäß Anspruch 1 oder Verwendung gemäß Anspruch 6 oder Antikörper nach Anspruch 7, wobei es sich bei dem Antikörper um einen polyklonalen, monoklonalen, chimären, humanisierten oder bispezifischen Antikörper oder ein epitopbindendes Fragment eines der Antikörper handelt.

## Revendications

1. Procédé de criblage et/ou de diagnostic d'un cancer des cellules rénales chez un sujet et/ou de suivi de l'efficacité d'une thérapie d'un cancer des cellules rénales, qui comprend l'étape de détection et/ou de quantification, dans un échantillon biologique obtenu à partir dudit sujet, d'un polypeptide CD27L, qui comprend ou qui est constitué par la séquence d'acides aminés présentée à la Figure 1 (SEQ ID n° : 2) ; dans lequel ladite étape comprend l'utilisation d'un anticorps qui se lie spécifiquement au polypeptide CD27L.

2. Procédé selon la revendication 1, dans lequel le taux dudit polypeptide est comparé avec une gamme de référence préalablement déterminée ou un témoin.

3. Procédé selon la revendication 1 ou la 2, dans lequel l'étape de détection comprend les étapes consistant à :
(a) mettre en contact l'échantillon avec un anticorps, tel que défini dans la revendication 1 ; et
(b) détecter l'apparition d'une éventuelle liaison entre l'anticorps et ledit polypeptide dans l'échantillon.

4. Procédé selon la revendication 3, dans lequel l'étape (b) comprend une détection du polypeptide capturé en utilisant un réactif de détection marqué directement ou indirectement.

5. Procédé selon la revendication 3 ou la 4, dans lequel l'anticorps est immobilisé sur une phase solide.

6. Utilisation d'un anticorps qui se lie spécifiquement à un CD27L dans la fabrication d'une composition destinée au traitement d'un cancer des cellules rénales, ledit anticorps étant conjugué à un agent, ou à une fraction de substance médicamenteuse, thérapeutique.

7. Anticorps, qui se lie spécifiquement à un CD27L, destiné à être utilisé dans le traitement d'un cancer des cellules rénales, ledit anticorps étant conjugué à un agent, ou à une fraction de substance médicamenteuse, thérapeutique.

8. Utilisation, selon la revendication 6, ou anticorps, selon la revendication 7, dans laquelle la fraction de substance médicamenteuse est une toxine.

9. Procédé, selon la revendication 1, utilisation, selon la revendication 6, ou anticorps, selon la revendication 7, dans lesquels l'anticorps est polyclonal, monoclonal, chimérique, humanisé ou bispécifique ou bien est un fragment de liaison à l'épitope de l'un quelconque de ceux-là.
